# EUROPEAN PATENT APPLICATION

(11) **EP 4 745 957 A1**
(43) Date of publication of application: **20.05.2026**
(21) Application number: 25214842.4
(22) Date of filing: 11.11.2025
(51) Int. Cl.: G10L 15/22, G16H 10/20, G10L 15/06, G16H 15/00

(54) **VOICE COMMAND ACTIVATED ASSISTANT TOOL FOR CLINICAL RESEARCH**

(30) Priority: 14.11.2024 US 202463720591 P; 26.02.2025 US 202519064228
(71) Applicant: Clinasyst LLC, Jacksonville, FL 32216 (US)
(72) Inventor: Koren, Michael J., Jacksonville (US)
(74) Representative: Greaves Brewster LLP

(57) **Abstract**

The invention relates to a clinical trials management system which includes a voice command activated assistant tool for clinical research. A unique training model is employed in which errors can be made in voice commands, such as voice/accent/background noise and the like, and the system will still route the voice command to the correct algorithm.

## Description

### CROSS REFERENCE TO RELATED APPLICATIONS

Priority is claimed on Provisional Patent Application Nos. 63/558,203, filed February 27, 2024, and 63/720,591, filed November 14, 2024, the contents of which are incorporated herein by reference.

### STATEMENT REGARDING FEDERALLY SPONSORED RESEARCH OR DEVELOPMENT

### REFERENCE TO A "SEQUENCE LISTING", A TABLE, OR A COMPUTER

### PROGRAM LISTING APPENDIX SUBMITTED ON COMPACT DISC

### BACKGROUND OF THE INVENTION

### 1. FIELD OF THE INVENTION

The present invention relates to Clinical Trials Management Systems (CTMS) and more particularly, to a computer-implemented system for a voice command activated assistant tool for clinical research which enhances medical provider productivity.

### 2. DESCRIPTION OF PRIOR ART INCLUDING INFORMATION DISCLOSED UNDER 37 CFR 1.97 AND 1.98

Information Technology (IT) and Artificial Intelligence (AI) applications have significantly impacted multiple industries. Unfortunately, for the conduct of contemporary medical practice, the new IT implementation has led to uneven results. For example, widespread adoption of electronic health records (EHR) and digital tracking systems (DTS), productivity in medical areas has not improved, and has become arguably worse for many settings in recent years. Medical quality metrics have also faltered despite widespread IT deployment.

This uninspiring track record for medical practice IT reflects several factors. Part of the problem involves information siloing, which prevents applications from working together effectively. Another problem arises because software development and design do not enhance the many established routines of physicians and other providers. Rather, medical industry IT usually requires the users to "retrain" and change longstanding, time-proven practice methods to coexist with the software. It in medicine has become the tail that wags the dog!

Clinical Trials Management Systems (CTMS) are software and database programs that help researchers conduct many elements of conducting clinical trials - such as scheduling, research protocol management, payment management for vendors and patients, subject recruiting, and regulatory document processing. These systems keep track of complex sets of research subject, staff, protocol and business data. Currently, these systems do not integrate the above resources with the day-today activities of physicians and other providers. To capture data or initiate a task that arises during patient interactions, the provider must stop what he or she is doing, get access to a computer and a software program, and then navigate that program. This disruptive process leads to reduced productivity and lower quality.

### BRIEF SUMMARY OF THE INVENTION

The present invention relates to a tool that enhances medical provider productivity through a unique integration of hardware, software and human assets that allows a provider to carry out specific and complex clinical research tasks using simple voice commands. A password-free, biometrically authenticated, experientially tested and validated, voice commanded device may be used to electronically sign regulatory and other critical documents for health applications using a paperless signature system.

### BRIEF DESCRIPTION OF SEVERAL VIEWS OF DRAWINGS

To these and to such other objects that may hereinafter appear, the present invention relates to clinical trials management systems and more particularly, to a voice command activated assistant tool for clinical research which employes a unique training model algorithm as described in detail in the following specification and recited in the annexed claims, taken together with the accompanying drawings in which:
Figure 1 is a flow chart illustrating the operation of the computer-implemented system of the present invention from the access of the voice command to the completion of the clinical research task;
Figure 2 is a flow chart showing an example of how the system performs a selected algorithm a specific task, such as Serious Adverse Event (SAE) report;
Figures 3A and 3B together illustrate the training model used by the system to accurately select the correct algorithm for a research task by eliminating errors which occur in a voice command.

### DETAILED DESCRIPTION OF THE INVENTION

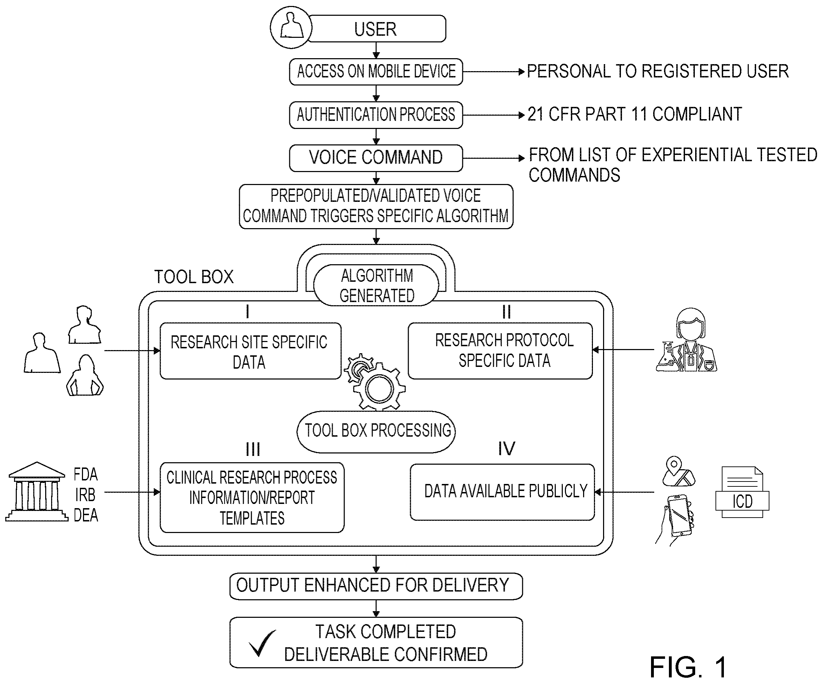

Figure 1 shows a flow chart of the workings of the tool. At top, the flow chart depicts a physician or staff member who identifies a task that requires action. These actions commonly include filing a regulatory report related to a patient interaction (such as a Serious Adverse Event Report), scheduling a visit or evaluation, providing information to a patient or colleague about a clinical trial, or recording an unscheduled visit for a subject enrolled in a research study.

The provider accesses the tool on his or her personal mobile device and gains access to the tool. The mobile device listens for instructions about the task that requires accomplishment.

The providers use a specific voice command that the system created to identify the task at hand. The tool has access to a prepopulated library of these tasks, previously established for robustness, that uniquely correlates a given voice command to a specific task.

This feature deploys "experiential testing". Experiential testing involved asking the system to interpret the voice command of many users with different accents and background noises (500 + for commands in our system). The system assesses what it "believes" the user said. The common "correct" interpretations and common "incorrect" interpretations are mapped to a single operating command of this system. Infrequent interpretation errors are discarded and the system asks the user to repeat the request.

Because all correct responses and frequent incorrect responses are uniquely mapped to a single command, the system will be robust (i.e. pick up most accents, etc), but not create an error related to one command being interpreted as another with a strong degree of confidence based on actual experience.

Once the system has accurately identified the task, the tool will direct the task to an algorithm to complete the task. Each task request will deploy a specific algorithm to act on the voice command. These algorithms integrate data and knowledge available to the system from one or more of the following: 1) the user; 2) the location of the user; 3) time and date of the request; 4) knowledge of the patient involved, if he or she has data in the system; 5) knowledge of the specific research process requested by the user; 6) knowledge of business data, if required to complete the task; 7) knowledge of the research project(s) that include the user and patient; 8) pre-designed forms, emails, texts and/or other correspondences associated with the request; 9) specifics of the research protocol at the specific location that involves the user and patient, if required; 10) knowledge of the regulatory agencies and sponsors who oversee the research project of the user and patient, if necessary to complete the task; 11) general internet available data needed to complete a report or the task as necessary; 12) An electronic signature application, if necessary to complete the task; 13) an e-commerce application if necessary, to complete the task.

Figure 1 shows the various assets that the tool can access for various specific algorithms related to clinical research operations. Figure 1 also depicts human interactions to enter or enable data input specific and proprietary to the task requested by the user. The tool uses a different pathway (i.e., algorithm) for each task, but accomplishing each task typically requires one or more of the following:
I. Research Site Specific Data - Information about the users and other staff members, the research portfolio (projects at a site) and patients. This system defines which users have permission to provide these data entries. The system also accommodates EHR sources of patient data.
II. Research Protocol Specific Data - Entered by staff trained to enter details of each specific research project or information transfer from research sponsors to the system (study protocol, vendor lists, research monitors, assigned institution review committee, etc.).
III. Clinical Research Process Information - System has captured knowledge of FDA and other governmental rules and regulations, standard forms, delegation of authority, and HIPAA requirements.
IV. Data available publicly - Geolocation, time, date, generally accepted diagnosis codes, etc.

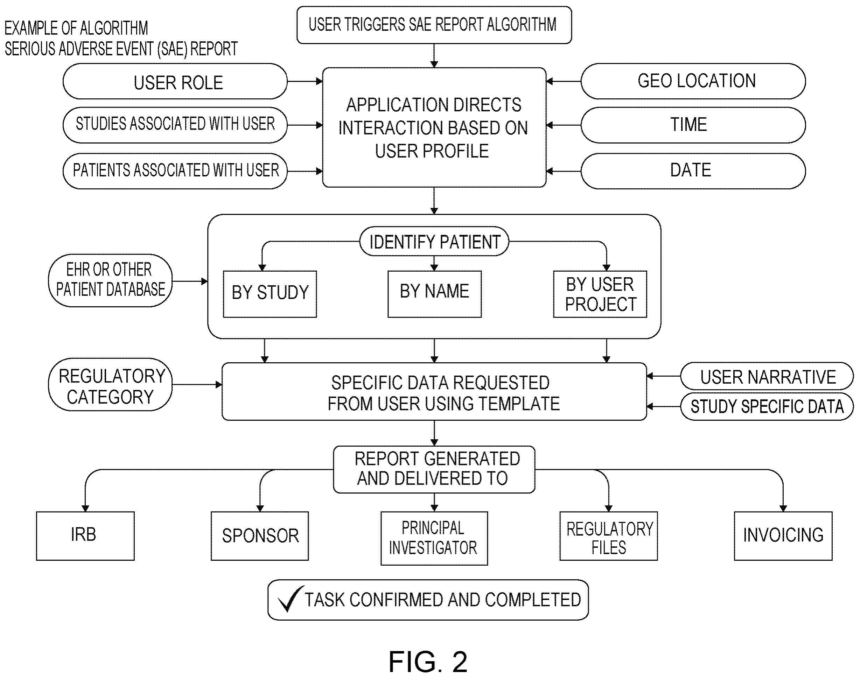

Figure 2 illustrates how the tool uses an algorithm for a specific research process. This figure details how the tool integrates multiple steps to create and complete a research report for a Serious Adverse Event (required by 21.CFR.312.32). All research investigators accept the responsibility of initiating and completing these reports. The reports are filed with the FDA, IRB and research sponsor within 24 hours of triggering events such as hospitalizations, life-threatening injuries, congenital anomalies, or death.

Currently, an investigator who sees a patient in the hospital, for example, has no immediate available means to generate or file these reports. Typically, this reporting involves 3 to 5 hours of manual report generation with patients, physicians, research coordinators, and other staff sending individual correspondences back and forth to compile all the data necessary for the report.

The current invention allows the user (physician or other health professional) at the time and point of contact with a patient (or during another medical interaction) to fully produce a report, file it with the appropriate parties and invoice for the pre-determined reimbursement for the service. The described tool will likely reduce the time taken for this task by > 90% and improve the accuracy of the reporting.

Other common applications for which the tool will markedly improve productivity through data system integrations include patient scheduling, messaging to groups affected by research findings (for example, informing all study patients about pertinent findings at the time study results become shared with the provider), logging unanticipated visits and findings during patient contact outside of the protocol specified visits, etc.

Another aspect of the present invention relates to a password-free, biometrically authenticated, experientially tested and validated, voice commanded device to electronically sign regulatory and other critical documents for health applications using a paperless signature system which is 21 CFR PART 11 Compliant.

Figure 2 shows one example of one of many specific algorithms within the system (SAE Report filing). Multiple voice commands each have their own algorithm mapped uniquely to avoid errors related to user speech accents or background sounds.

The system takes complex voice instruction and limits the algorithm pathway to improve accuracy. The voice recognition doesn't have to be perfect to get to the unique and appropriate algorithm as the system easily identifies this task for the user.

A novel aspect of the invention involves the robustness of the algorithm direction. Most voice recognition tools make mistakes due to accents, background noise etc. The voice commands in the invention are built for robustness meaning that many errors in the voice recognition will still lead to the proper addressing of the command to the algorithm.

The actual algorithm involves specific and intimate knowledge of how processes work at a clinical research site. So given the example of an SAE report, the system will pull data from different sources to produce the final deliverable. In the algorithms there are some proprietary elements which will be unique to each process (business processes specific for each company that uses the system). A novelty of this invention relates to the way the voice commands are routed to the algorithms.

The system allows this flexibility, ease of use, and responsiveness to the circumstances of running clinical trials and efficiently complying with regulatory requirements.

Errors can be made in voice commands and still the system will get to the correct algorithm. Each algorithm is unique. The system is a way to route to each algorithm that eliminates mistakes from voice/accent/background noise. The novelty is in the way the voice command is routed to the algorithm.

The following is the output report from our Experience Testing. The voice command of "SAE Report" tested over 500 times with different people in different settings. The data attached shows all commands the system heard more than once, and each of these is now routed to "SAE Command". This "experiential testing" overcomes previous barriers of this type of technology (i.e. what people say and what computers hear are different, especially in diverse individuals and settings). With this technology, the algorithm maps mistakes to the correct command which will never overlap with any other commands, creating great precision for key commands. This embedded in a broader system delivers a product that quickly accomplishes a medical task that was not done before this and also was not feasible due to the concern of inaccuracies.

| **WORD** | **COUNT** |
|---|---|
| SAE report | 282 |
| Essay report | 29 |
| SAT report | 28 |
| Yes a E report | 12 |
| I see you report | 6 |
| sorry report | 5 |
| Essay report a | 5 |
| SAE reports | 5 |
| Say a report | 4 |
| SAE | 4 |
| I see your report | 4 |
| Say report | 3 |
| essay | 3 |
| SA E report | 3 |
| Yes AE report | 3 |
| As a airport | 2 |
| they say you report | 2 |
| Yes a year report | 2 |
| Sayre report | 2 |
| yes I report | 2 |
| side report | 408 |

The system training model is designed to address the inaccuracies often found in traditional voice recognition systems. The model achieves a high level of accuracy through our unique training protocol. To train the model, a smartphone is taken to various locations to gather voice samples from approximately 500 different individuals as they pronounce the intended system voice commands.

The actual transcriptions from the phone are recorded and the data analyzed (Fig. 1). If the intended term ranks as the most frequently recognized on the distribution list, the system retains it and associates it with the corresponding action. For instance, if "SAE report" is the top term, it will be linked to the action of filing an SAE report. In this scenario, all common errors (i.e. > 1 error), such as "Essay Report," "SAT Report," or "Yes a E Report," etc. are categorized under the action of submitting a serious adverse event report. All uncommon errors (<1) are discarded and not coded to the selected term.

Alternatively, if the intended term does not rank highest, as seen when "SAE report" ranks third on the nomogram, the algorithm will discard the term "SAE Report" and select a different term for the command. In such a case, the "Serious Adverse Event Report" might be selected as the chosen term. The system then re-evaluates the nomogram with this new term "Serious Adverse Event", and if it emerges as the most frequently heard, the system would accept it as the official command for submitting a Serious Adverse Event Report. The system would subsequently code all common errors (i.e. more than 1 frequency) to "Serious Adverse Event Report" and all uncommon errors would be discarded from the system.

| Word | Count |
|---|---|
| Essay Report | 275 |
| Es Report | 75 |
| SAE Report | 50 |
| Sorry Report | 25 |
| I See you Report | 25 |
| Say a Report | 25 |
| I See you Report | 25 |

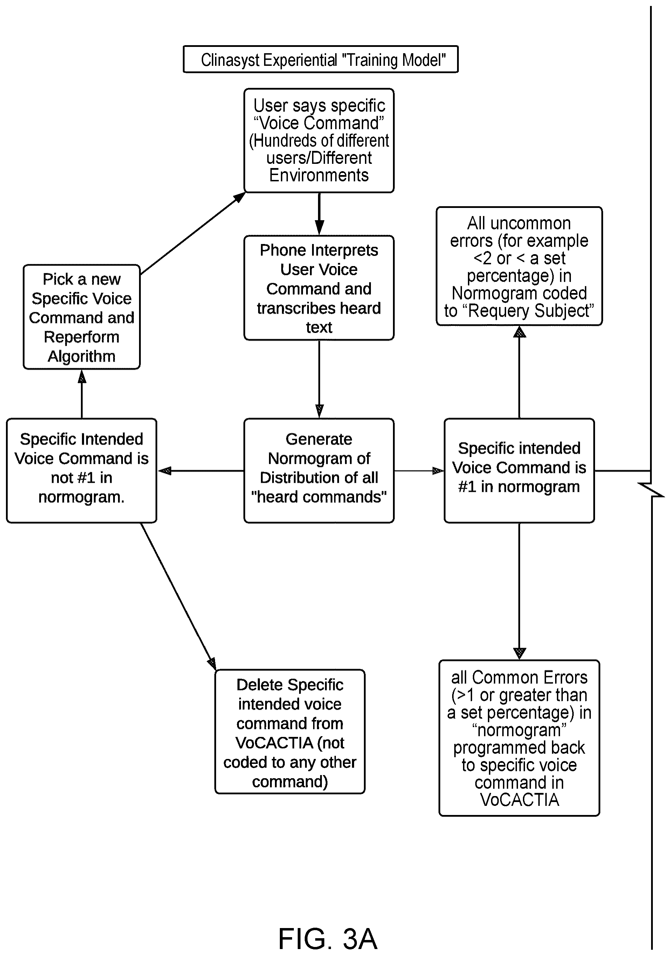

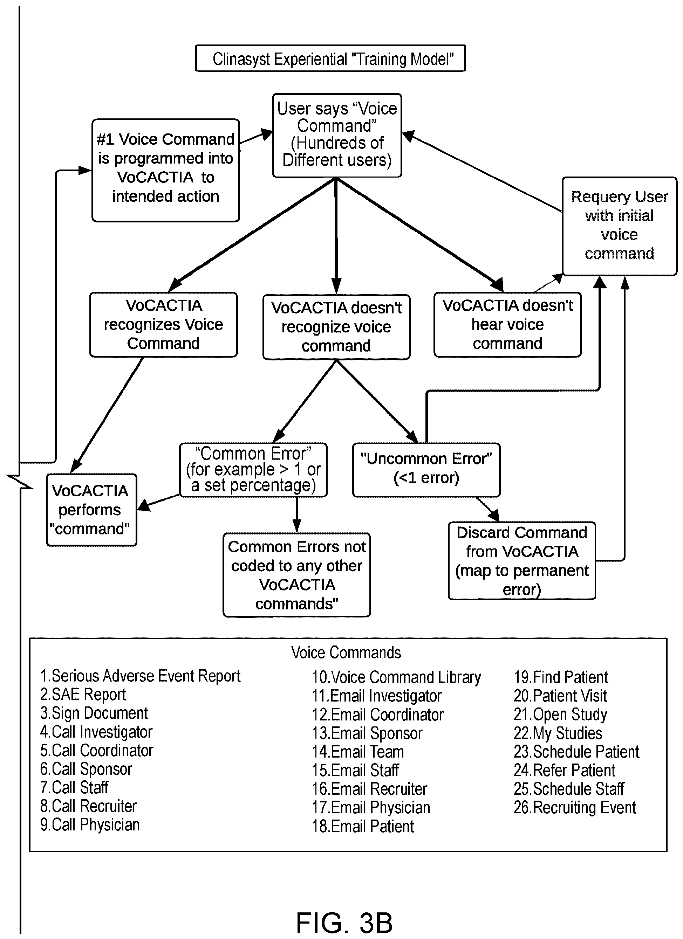

Figures 3A and 3B together illustrate in more detail how the algorithm is trained to increase accuracy by recognizing and deleting errors. As seen in Figure 3A, when the user says a voice command into a phone, the system interprets the user's voice command and transcribes the heard text. A nomogram of Distribution of all "heard commands" is generated.

If the specific intended voice command is not #1 in the nomogram, the specific voice command will be deleted from the voice command library and will not be coded to any other command. A new specific voice command is picked. The algorithm is reperformed and the user chooses a new specific voice command chosen by voice.

The new specific voice command is interpreted, transcribed and a nomogram of distribution of all "heard commands" is generated. If the specific intended voice command is #1 in the nomogram, all common errors (less than 1) in the nomogram are coded to "requery subject" and all common errors (greater than1) in the nomogram are programmed back to a specific voice command in the voice command library.

In Figure 3B, if no errors are found in the nomogram, the #1 voice command is programed into the system to intended action. The user says a voice command and the system either recognizes the voice command, does not recognize the voice command or does not hear the voice command. If the voice command is not heard, the system will requery the user with the initial voice command.

If the system recognizes the voice command, the command is performed. If the system does not recognize the voice command, a common error (greater than 1) is noted and the system either performs the command or the common error is not coded to any other command in the voice command library. If an uncommon error (less than 1) is noted, the system will requery the user with the initial voice command or the command is discarded from the voice command library and mapped to permanent error. In that case, the system will requery the user with the initial voice command.

At the bottom of Figure 3B a sample list of possible voice commands in the voice command library is provided
While only a limited number of preferred embodiments of the present invention have been disclosed for purposes of illustration, it is obvious that many modifications and variations could be made thereto. It is intended to cover all of those modifications and variations which fall within the scope of the present invention, as defined by the following claims.

## Claims

1. A computer-implemented system for a voice command activated assistant tool for clinical research which allows a medical provider (user) to perform specific and complex clinical research tasks using simple voice commands, said system comprising the steps of creating an algorithm by:
taking a smartphone to various locations to gather voice samples from multiple individuals as they pronounce the intended system voice commands;
recording the actual transcriptions from the phone and analyzed data;
receiving a voice command;
generating a nomogram of distribution of all "heard commands;"
retaining the voice command and if voice command is most frequently recognized on the distribution list of the nomogram:
(i) associating retained voice command with the corresponding action; or
(ii) programming all common errors in the retained voice command back to specific voice command in system; or
(iii) coding all uncommon errors in the retained voice command to "requery subject."

2. The system of claim 1 further comprising the steps of; deleting the intended voice command from system if the retained voice command is not most frequently recognized on the distribution list of the nomogram or picking a new voice command and reperforming the algorithm if the retained voice command is not the most frequently recognized on the distribution list of the nomogram.

3. The system of claim 1 wherein retained voice command associated with the corresponding action is recognized by system and performs task.

4. The system of claim 1 wherein system does not recognize voice command because of common error, system performs command or is not coded to any other command.

5. The system of claim 1 wherein system does not recognize voice command because of uncommon error, system requeries user initial command or discards voice command and requeries user initial command.

6. The system of claim 1 wherein system does not hear the voice command, system requeries user initial voice command.

7. A computer-implemented system for training an algorithm in a voice command activated assistant tool for clinical research to correlate a voice command with a specific task comprising the steps of:
taking a smartphone to various locations to gather voice samples from multiple individuals as they pronounce the intended system voice commands;
recording the actual transcriptions from the phone and analyzed data;
receiving a voice command;
generating a nomogram of distribution of all "heard commands;"
retaining the voice command and if voice command is most frequently recognized on the distribution list of the nomogram:
(i) associating retained voice command with the corresponding action; or
(ii) programming all common errors in the retained voice command back to specific voice command in system; or
(iii) coding all uncommon errors in the retained voice command to "requery subject."

8. The system of claim 7 further comprising the steps of; deleting the intended voice command from system if the retained voice command is not most frequently recognized on the distribution list of the nomogram or picking a new voice command and reperforming the algorithm if the retained voice command is not the most frequently recognized on the distribution list of the nomogram.

9. The system of claim 7 wherein retained voice command associated with the corresponding action is recognized by system and performs task.

10. The system of claim 7 wherein system does not recognize voice command because of common error, system performs command or is not coded to any other command.

11. The system of claim 7 wherein the system does not recognize voice command because of uncommon error, system requeries user initial command or discards voice command and requeries user initial command.

12. The system of claim 7 wherein system does not hear the voice command, system requeries user initial voice command.

13. The system of claim 7 further comprising the steps of;
deleting the intended voice command from system if intended voice command is not most frequently recognized on the distribution list of the nomogram; or picking a new voice command and reperforming the algorithm is not most frequently recognized on the distribution list of the nomogram.

14. The system of claim 7 wherein voice command is recognized by system and performs task.

15. The system of claim 1 wherein the algorithm is generated using one or more of the following:
Research Protocol Specific Data - Entered by staff trained to enter details of each specific research project or information transfer from research sponsors to the system (study protocol, vendor lists, research monitors, assigned institution review committee,
Clinical Research Process Information - System has captured knowledge of FDA and other governmental rules and regulations, standard forms, delegation of authority, and HIPAA requirements.
Data available publicly - Geolocation, time, date, generally accepted diagnosis codes.

16. The system of claim 1 comprising an algorithm which integrates data and knowledge available to the system from one or more of the following:
1) the user; 2) the location of the user; 3) time and date of the request;
4) knowledge of the patient involved, if he or she has data in the system; 5) knowledge of the specific research process requested by the user; 6) knowledge of business data, if required to complete the task;
7) knowledge of the research project(s) that include the user and patient; 8) pre-designed forms, emails, texts and/or other correspondences associated with the request; 9) specifics of the research protocol at the specific location that involves the user and patient, if required; 10) knowledge of the regulatory agencies and sponsors who oversee the research project of the user and patient, if necessary to complete the task; 11) general internet available data needed to complete a report or the task as necessary; 12) An electronic signature application, if necessary to complete the task; 13) an e-commerce application if necessary, to complete the task.

17. The system of claim 7 wherein the algorithm is generated using one or more of the following:
Research Protocol Specific Data - Entered by staff trained to enter details of each specific research project or information transfer from research sponsors to the system (study protocol, vendor lists, research monitors, assigned institution review committee,
Clinical Research Process Information - System has captured knowledge of FDA and other governmental rules and regulations, standard forms, delegation of authority, and HIPAA requirements.
Data available publicly - Geolocation, time, date, generally accepted diagnosis codes.

18. The system of claim 7 comprising an algorithm which integrates data and knowledge available to the system from one or more of the following:
1) the user; 2) the location of the user; 3) time and date of the request;
4) knowledge of the patient involved, if he or she has data in the system; 5) knowledge of the specific research process requested by the user; 6) knowledge of business data, if required to complete the task;
7) knowledge of the research project(s) that include the user and patient; 8) pre-designed forms, emails, texts and/or other correspondences associated with the request; 9) specifics of the research protocol at the specific location that involves the user and patient, if required; 10) knowledge of the regulatory agencies and sponsors who oversee the research project of the user and patient, if necessary to complete the task; 11) general internet available data needed to complete a report or the task as necessary; 12) An electronic signature application, if necessary to complete the task; 13) an e-commerce application if necessary, to complete the task.
